Europäisches Patentamt

European Patent Office

Office européen des brevets

(11) Veröffentlichungsnummer: **0 022 469**
**B1**

(12) **EUROPÄISCHE PATENTSCHRIFT**

(45) Veröffentlichungstag der Patentschrift:
02.03.83

(51) Int. Cl.³: **C 07 D 307/08**

(21) Anmeldenummer: **80103070.1**

(22) Anmeldetag: **03.06.80**

(54) Verfahren zur Abtrennung von Methanol aus Mischungen von Tetrahydrofuran mit Methanol und Wasser.

(30) Priorität: **11.07.79 DE 2927931**

(43) Veröffentlichungstag der Anmeldung:
**21.01.81 Patentblatt 81/3**

(45) Bekanntmachung des Hinweises auf die Patenterteilung:
**02.03.83 Patentblatt 83/9**

(84) Benannte Vertragsstaaten:
**BE FR GB IT NL**

(56) Entgegenhaltungen:
**FR-A-2 340 314**
**GB-A-1 402 507**

(73) Patentinhaber: **CHEMISCHE WERKE HÜLS AG,
Postfach 1320, D-4370 Marl 1 (DE)**

(72) Erfinder: **Müller, Wolfgang Hans Eduard, Dr., Bitterfelder
Strasse 9a, D-4370 Marl (DE)**
Erfinder: **Zölffel, Michael, Höchster Strasse 20,
D-4370 Marl (DE)**

**0 022 469**

### Verfahren zur Abtrennung von Methanol aus Mischungen
### von Tetrahydrofuran mit Methanol und Wasser

Die Erfindung betrifft ein Verfahren zur kontinuierlichen Abtrennung von Methanol aus Mischungen, die im wesentlichen Tetrahydrofuran und daneben Methanol, Wasser und gegebenenfalls weitere Stoffe enthalten.

Zweck der Erfindung ist die Gewinnung reinen Tetrahydrofurans, das insbesondere kein Wasser oder Methanol enthält, wobei aus wirtschaftlichen Gründen nur geringe Verluste an Tetrahydrofuran hingenommen werden können.

Tetrahydrofuran wird z. B. aus Butandiol mit Schwefelsäure oder sauren Ionenaustauschern als Katalysator hergestellt. Die Reaktionsprodukte bestehen hauptsächlich aus Tetrahydrofuran und Wasser neben kleineren Anteilen weiterer Stoffe, wobei auch Methanol anwesend sein kann. Bekannterweise ist Wasser mit Tetrahydrofuran vollständig mischbar und außerdem bilden diese Stoffe ein Minimumazeotrop. Das normalerweise zur Entwässerung organischer Flüssigkeiten, die mit Wasser Minimumazeotrope bilden, angewendete Verfahren der Rektifikation in zwei Kolonnen, wobei die Kopfprodukte beider Kolonnen azeotrope Zusammensetzung haben und nach Kondensation in einer Trennflasche in zwei flüssige Phasen getrennt werden, ist daher zur Trocknung von Tetrahydrofuran nicht geeignet, weil sich das Minimumazeotrop nicht in zwei flüssige Phasen trennt. Diese Schwierigkeit wurde bekannterweise dadurch überwunden, daß man die Trennung in zwei flüssige Phasen durch Zusatz eines dritten Stoffes herbeiführte und die getrennten flüssigen Phasen entsprechend rektifizierte. Weiterhin werden für die Trennung von Wasser und Tetrahydrofuran die extraktive Destillation, Absorptionsverfahren oder Methoden benutzt, die auf chemischer Reaktion beruhen. Eine besonders elegante Methode zur Entwässerung von Tetrahydrofuran ist die Rektifikation in zwei bei unterschiedlichen Drücken betriebenen Kolonnen, bei der die Kopfprodukte einer Kolonne der jeweils anderen Kolonne wieder zugeführt werden und aus den Kolonnensümpfen die getrennten Stoffe Wasser und Tetrahydrofuran entnommen werden (DE-AS 1 901 870).

In der Patentschrift GB-1 402 507 wird die Reinigung von Gemischen beschrieben, die Tetrahydrofuran und Methanol enthalten. Dabei wird das am Kopf der zweiten Kolonne abgezogene methanolhaltige Destillat in die erste Kolonne zurückgeführt. Das vom Kopf der ersten Kolonne abgezogene Destillat wird ausgeschleust; es enthält eine erhebliche Menge an Tetrahydrofuran. Zur Gewinnung dieses Anteils wird weiter vorgeschlagen, das Methanol mit Säuren zu verestern und das Tetrahydrofuran in einer weiteren Rektifikation teilweise zurückzugewinnen. Die vollständige Abtrennung des Methanols am Kopf der ersten Kolonne ist schwierig, wenn dieses Methanol möglichst wenig Tetrahydrofuran enthalten soll, selbst dann, wenn man ein System aus drei Kolonnen einsetzt.

Die genannten Verfahren führen zu Schwierigkeiten, falls mehr als etwa 0,01% Methanol in dem aufzuarbeitenden wäßrigen Tetrahydrofuran enthalten ist. Bisher sind keine Methoden bekanntgeworden, mit denen man methanolhaltiges Tetrahydrofuran von Wasser und Methanol befreien und in hoher Ausbeute und hoher Reinheit isolieren kann. Derartige methanolhaltige Rohtetrahydrofurane fallen an, wenn z. B. anstelle des relativ teuren Reinbutandiol-1,4 das aus der Polyesterherstellung stammende unreine Butandiol-1,4 als Rohstoff für die Synthese von Tetrahydrofuran mit Schwefelsäure als Katalysator eingesetzt wird.

Es stellt sich daher die Aufgabe, eine Methode zu finden, die es gestattet, aus derartigen Tetrahydrofuran, Wasser, Methanol und gegebenenfalls weitere Stoffe enthaltenden Gemischen das Methanol abzutrennen und in möglichst hoher Ausbeute reines, d. h. den üblichen Handelsqualitäten entsprechendes, Tetrahydrofuran zu gewinnen, das praktisch frei von Methanol und Wasser ist.

Die erfindungsgemäße Lösung der Aufgabe wurde darin gefunden, daß man das methanolhaltige feuchte Tetrahydrofuran in einem System von mindestens zwei Kolonnen ohne weitere Zusätze rektifiziert, und zwar in der Weise, daß die beiden Kolonnen unter verschiedenem Druck betrieben werden, wobei das Destillat aus der unter geringerem Druck betriebenen Kolonne der unter höherem Druck betriebenen Kolonne zugeführt wird und aus dem Abtriebs- oder Verstärkerteil der unter höherem Druck betriebenen Kolonne ein Seitenstrom entnommen wird, der in die unter geringerem Druck betriebene Kolonne zurückgeführt wird. Am Kopf der unter höherem Druck betriebenen Kolonne wird ein methanolreiches, wasserarmes Produkt entnommen, das praktisch das gesamte mit dem Rohstoff in die Rektifiziereinrichtung eingebrachte Methanol enthält. Im Sumpf der unter geringerem Druck betriebenen Kolonne fällt tetrahydrofuranfreies Wasser und im Sumpf der unter höherem Druck betriebenen Kolonne wasser- und methanolfreies Tetrahydrofuran an. Das wasser- und methanolfreie Tetrahydrofuran kann — falls erforderlich — in einer dritten Rektifikationskolonne in bekannter Weise von höhersiedenden Verunreinigungen, wie z. B. Methyltetrahydrofuran, befreit werden. Die Skizze, aus der alle notwendigen, jedoch nicht erfindungsspezifischen weiteren Vorrichtungen, wie z. B. Pumpen und Druckminderventile, weggelassen wurden, verdeutlicht das erfindungsgemäße Verfahren:

Der Stoffstrom (4), der neben Tetrahydrofuran, Wasser und Methanol noch weitere Stoffe, wie z. B. Butandiol-1,4 oder 3-Methyltetrahydrofuran, enthalten kann, wird einer kontinuierlich betriebenen Rektifikationskolonne (1) zugeleitet. Derartige Gemische fallen z. B. bei der Synthese von

2

Tetrahydrofuran aus Butandiol-1,4 mit sauren Katalysatoren an, insbesondere dann, wenn Rohstoffe eingesetzt werden, die neben Butandiol-1,4 noch andere Stoffe (z. B. Methylester) enthalten. Bei der Rektifikationskolonne (1) handelt es sich um eine Kolonne üblicher Bauart, also z. B. eine Glockenbodenkolonne, Ventilbodenkolonne oder Füllkörperkolonne, die bei einem Druck, der deutlich niedriger als der Druck in Kolonne (2) ist, betrieben wird; der Druck in der Kolonne (1) kann der Normaldruck sein. Je nach Konzentration des Stoffstroms (4) sind 10 bis 30 theoretische Trennstufen und Rücklaufverhältnisse zwischen 0,5 und 4 zweckmäßig. Aus dem Sumpf der Kolonne (1) wird das im Rohstoff enthaltene Wasser — praktisch tetrahydrofuranfrei — entnommen [Stoffstrom (5)]. Dieser Stoffstrom (5) kann auch noch andere im Rohstoff gegebenenfalls anwesende Stoffe enthalten, wie z. B. Butandiol-1,4. Mit dem Stoffstrom (6) wird das gesamte der Kolonne (1) zugeführte Tetrahydrofuran, das gesamte der Kolonne (1) zugeführte Methanol, ein Teil des der Kolonne (1) zugeführten Wassers und gegebenenfalls noch weitere anwesende Stoffe, wie z. B. 3-Methyltetrahydrofuran, der Kolonne (2) zugeführt. Da die Kolonne (2) unter einem höheren Druck als die Kolonne (1) (z. B. unter einem Druck zwischen 3 und 25 bar, bevorzugt zwischen 10 und 15 bar) betrieben wird, muß der Stoffstrom (6) mittels einer Pumpe der Kolonne (2) zugeleitet werden. Der Stoffstrom (6) kann in die Kolonne (2) sowohl oberhalb der Seitenstromentnahme als auch unterhalb der Seitenstromentnahme eingeleitet werden. Die Kolonne (2) ist eine Druckkolonne üblicher Bauart und sollte etwa 15 bis 50 theoretische Stufen aufweisen. Am Kopf der Kolonne (2) wird das gesamte mit Stoffstrom (4) zugeführte Methanol in Form eines methanolreichen, wasserarmen Stoffstroms (7) entnommen. Entweder aus der Verstärkungssäule oder aus der Abtriebssäule wird dampfförmig oder flüssig ein Seitenstrom (8) entnommen, der praktisch das gesamte der Kolonne (2) mit Stoffstrom (6) zugeführte Wasser enthält, und der zum mittleren Teil der Kolonne (1) zurückgeleitet wird. Dem Sumpf der Kolonne (2) wird der Stoffstrom (9) entnommen, der mit Ausnahme der geringen in Stoffstrom (7) enthaltenen Tetrahydrofuranmenge praktisch die gesamte der Trennanlage mit Stoffstrom (4) zugeführte Tetrahydrofuranmenge enthält. Dieser Stoffstrom (9) kann, sofern dies erforderlich ist, in einer Kolonne (3) in ein besonders reines Tetrahydrofuran [Stoffstrom (10)] und einen praktisch tetrahydrofuranfreien Stoffstrom (11) zerlegt werden, der höher als Tetrahydrofuran siedende Stoffe, wie z. B. 3-Methyltetrahydrofuran, enthält. Die Kolonne (3) hat z. B. 15 bis 40 theoretische Stufen und wird mit Rücklaufverhältnissen zwischen 0,5 und 4 betrieben.

Die Stoffströme (5), (7) und (11) können auf andere darin enthaltene Stoffe aufgearbeitet werden, oder sie werden umweltfreundlich vernichtet. Stoffstrom (7) kann auch als Lösemittel eingesetzt werden.

Die Vorteile des erfindungsgemäßen Verfahrens bestehen darin, daß man auch methanolhaltige Rohtetrahydrofurane in hoher Ausbeute zu besonders reinem Tetrahydrofuran aufarbeiten kann und daß man dies ohne weitere in der Investition und im Betrieb aufwendige Apparate erreicht. Der einzige erforderliche Mehraufwand ist die Verlängerung der in an sich bekannter Weise betriebenen Entwässerungszone in der unter höherem Druck betriebenen Kolonne (2). Ein zusätzlicher Energieaufwand ist nicht erforderlich, da die Verdampfung im Sumpf der unter höherem Druck betriebenen Kolonne ohnehin durchgeführt werden muß. Ohne die Verlängerung der unter höherem Druck betriebenen Kolonne und Abnahme eines methanolreichen Produkts am Kopf dieser Kolonne würde sich bei Verarbeitung eines methanolhaltigen Rohtetrahydrofurans innerhalb kurzer Zeit Methanol an den oberen Enden der beiden Entwässerungskolonnen ansammeln, und es würde kein weiteres Wasser mehr abgetrennt.

Das erfindungsgemäße Verfahren ist eine wesentliche Verbesserung des Standes der Technik, da nunmehr auch andere Rohstoffe, die wesentlich billiger als reines Butandiol-1,4 sind, in hoher Ausbeute in Tetrahydrofuran übergeführt werden können, ohne daß dadurch andere Nachteile in Kauf genommen werden müßten. Neben dem technischen ist das ein wesentlicher wirtschaftlicher Vorteil.

Das erfindungsgemäße Verfahren wird durch folgende Beispiele erläutert:

Beispiel 1

Eine Mischung aus 24,1 Gewichtsprozent Wasser, 1,95 Gewichtsprozent Methanol und 73,95 Gewichtsprozent Tetrahydrofuran wurde in zwei Rektifikationskolonnen kontinuierlich rektifiziert. Die erste Kolonne hatte 30 praktische Böden, der genannte Rohstoff wurde flüssig mit Siedetemperatur auf den sechsten Boden von unten gezählt geleitet und der aus der zweiten Rektifikationskolonne entnommene Seitenstrom wurde dem siebten Boden von unten zugeleitet. Die erste Kolonne wurde unter Normaldruck mit einem Rücklaufverhältnis von eins betrieben. Die zweite Kolonne war eine Füllkörperkolonne von 4 m Füllkörperschüttungshöhe, die unter einem Druck von 10 bar (abs) ebenfalls mit einem Rücklaufverhältnis von eins betrieben wurde. Das Destillat der ersten Kolonne wurde bei 1,5 m Füllkörperschüttungshöhe zugeführt, während der Seitenstrom in 2,5 m Höhe flüssig abgezogen wurde. Hierbei wurde die Hälfte der an dieser Stelle durch die Kolonne strömenden Flüssigkeit entnommen. Am Kopf der zweiten Kolonne mit einem Rücklaufverhältnis von 70 wurden 2,7 Gewichtsprozent [bezogen auf den in Kolonne (1) eingespeisten Rohstoff] eines Produktes mit 0,02 Gewichtsprozent Wasser, 70,6 Gewichtsprozent Methanol und 29,4 Gewichtsprozent Tetrahydrofuran

0 022 469

entnommen. Während im Sumpf der zweiten Kolonne 73,2 Gewichtsprozent [bezogen auf den in Kolonne (1) eingespeisten Rohstoff] mit einem Gehalt von >99,9 Gewichtsprozent Tetrahydrofuran und Wasser- bzw. Methanolgehalten von jeweils unter 0,05 Gewichtsprozent anfielen, wurden im Sumpf der ersten Kolonne 24,1 Gewichtsprozent [bezogen auf den in Kolonne (1) eingespeisten Rohstoff] entnommen, die weniger als 0,05 Gewichtsprozent Tetrahydrofuran enthielten.

Bezieht man alle Teilmengen auf die Menge des eingesetzten Rohstoffs, dann ergibt sich:

| Stoff- strom Nr. | Teilmengen bez. auf Rohstoffmenge = 100 Gew.-% | | | |
|---|---|---|---|---|
| | Tetrahydrofuran | Methanol | Wasser | Summe |
| 4 | 73,95 | 1,95 | 24,1 | 100 |
| 5 | <0,01 | <0,02 | 24,07 | 24,1 |
| 7 | 0,80 | 1,90 | 0,001 | 2,70 |
| 9 | >73,14 | <0,03 | <0,03 | 73,2 |

In diesem Beispiel sind die Temperaturen in den Kolonnen:

| | Kolonne 1 | Kolonne 2 |
|---|---|---|
| Kopf | 64°C | 137°C |
| Sumpf | 101°C | 162°C |

## Beispiel 2

Ein Rohtetrahydrofuran folgender Zusammensetzung:

| 26,3 Gew.-% | Wasser |
|---|---|
| 0,83 Gew.-% | Methanol |
| 71,0 Gew.-% | Tetrahydrofuran |
| 1,87 Gew.-% | andere Stoffe |

das aus einem sog. Rückbutandiol-1,4 aus der Polyesterherstellung gewonnen worden war, wurde in einer Apparatur aus drei Rektifikationskolonnen kontinuierlich rektifiziert. Die erste Kolonne hatte 30 praktische Böden, Rohstoffzufuhr flüssig mit Siedetemperatur auf den sechsten Boden von unten gezählt, Rückführung des Seitenstroms von Kolonne (2) auf den siebten Boden von unten gezählt. Die Kolonne wurde unter Normaldruck mit einem Rücklaufverhältnis von eins betrieben.

Die zweite Kolonne war eine Füllkörperkolonne mit 4 m Füllkörperschüttung, wobei das Destillat der ersten Kolonne in 3 m Füllkörperhöhe der zweiten Kolonne zugeleitet wurde. Der Seitenstrom wurde in 2,5 m Füllkörperschüttungshöhe abgezogen, wobei die Hälfte der in dieser Höhe durch die Kolonne strömenden Flüssigkeit als Seitenstrom entnommen und wieder der ersten Rektifikationskolonne zugeleitet wurde. Am Kolonnenkopf wurde mit einem Rücklaufverhältnis von 80 ein methanolreiches Destillat entnommen. Das Sumpfprodukt dieser zweiten Rektifikationskolonne wurde einer unter Normaldruck betriebenen dritten Rektifikationskolonne mit im ganzen 45 praktischen Böden auf den fünfundzwanzigsten Boden von unten gezählt zugeleitet. Diese dritte Rektifikationskolonne wurde mit einem Rücklaufverhältnis von drei betrieben. Bezogen auf den der ersten Kolonne zugeleiteten Rohstoff fielen folgende Mengen an:

| 26,9% | Sumpf Kolonne (1) |
|---|---|
| 1,35% | Destillat Kolonne (2) |
| 70,6% | Destillat Kolonne (3) |
| 1,15% | Sumpf Kolonne (3) |

Das Sumpfprodukt der ersten Kolonne war Wasser mit kleineren Anteilen anderer Stoffe. Der Tetrahydrofurangehalt war <0,05 Gewichtsprozent.

4

**0 022 469**

Das Destillat der zweiten Kolonne enthielt 61,2 Gewichtsprozent Methanol, 0,8 Gewichtsprozent Wasser, 33,9 Gewichtsprozent Tetrahydrofuran und kleinere Mengen anderer Stoffe.

Das Destillat der dritten Kolonne war Tetrahydrofuran mit einem Gehalt von > 99,9 Gewichtsprozent.

Das Sumpfprodukt der dritten Kolonne waren höhersiedende Verbindungen. Der Tetrahydrofurangehalt betrug < 0,05 Gewichtsprozent.

Bezieht man alle Teilmengen auf die Menge des eingesetzten Rohstoffs, dann ergibt sich:

| Stoff-strom Nr. | Teilmengen bezogen auf Rohstoffmenge = 100 Gew.-% | | | | |
|---|---|---|---|---|---|
| | Tetra-hydrofuran | Methanol | Wasser | Andere Stoffe | Summe |
| 4 | 71,0 | 0,83 | 26,3 | 1,87 | 100 |
| 5 | <0,01 | <0,01 | 26,24 | 0,64 | 26,9 |
| 7 | 0,48 | 0,80 | 0,02 | 0,05 | 1,35 |
| 10 | >70,5 | <0,02 | <0,04 | <0,04 | 70,6 |
| 11 | >0,001 | <0,001 | <0,001 | >1,14 | 1,15 |

Der Druck in Kolonne 2 beträgt 10 bar abs. Die Temperaturen sind:

| | Kolonne 1 | Kolonne 2 | Kolonne 3 |
|---|---|---|---|
| Kopf | 64°C | 137°C | 67°C |
| Sumpf | 101°C | 162°C | 117°C |

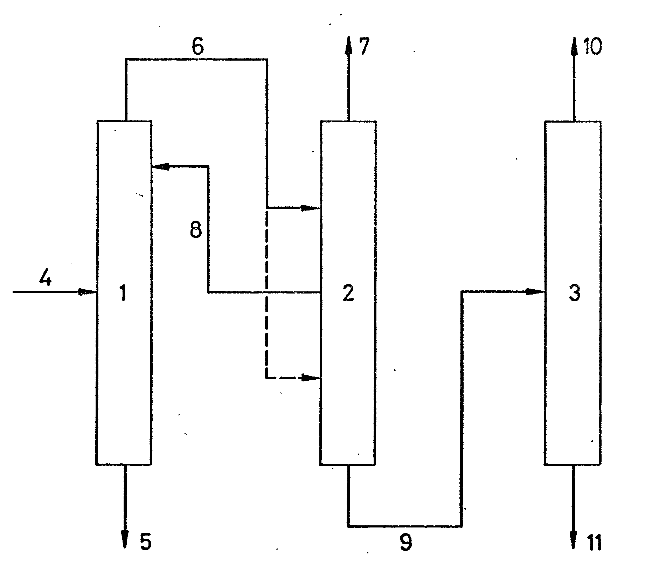

## Patentansprüche

1. Verfahren zur kontinuierlichen Abtrennung von Methanol aus Mischungen, die im wesentlichen Tetrahydrofuran und daneben Wasser, Methanol sowie gegebenenfalls weitere Stoffe enthalten, in einer Rektifiziereinrichtung aus mindestens zwei Rektifizierkolonnen, von denen zwei bei unterschiedlichen Drücken betrieben werden und das Destillat aus der unter geringerem Druck betriebenen Kolonne der unter höherem Druck betriebenen Kolonne zugeleitet wird, während ein Stoffstrom aus der unter höherem Druck betriebenen Kolonne in die unter geringerem Druck betriebene Kolonne zurückgeleitet wird, dadurch gekennzeichnet, daß der aus der unter höherem Druck betriebenen Kolonne entnommene Stoffstrom als Seitenstrom aus dem Abtriebs- oder Verstärkungsteil entnommen und in die unter geringerem Druck betriebene Kolonne zurückgeführt wird, während am Kopf der unter höherem Druck betriebenen Kolonne ein methanolreicher Stoffstrom, der praktisch das gesamte mit dem Rohstoff in die Rektifiziereinrichtung eingebrachte Methanol enthält, abgezogen wird.

2. Verfahren nach Anspruch 1, dadurch gekennzeichnet, daß die erste Kolonne unter Normaldruck und die zweite Kolonne unter einem Druck zwischen 3 und 25 bar, vorzugsweise zwischen 10 und 15 bar, betrieben wird.

3. Verfahren nach Anspruch 1 oder 2, dadurch gekennzeichnet, daß ein Rohtetrahydrofuran eingesetzt wird, das durch säurekatalysierte Umsetzung aus unreinem Butandiol-1,4 erhalten wurde.

## Claims

1. A process for the continuous separation of methanol from a mixture which contains primarily tetrahydrofuran and additionally water, methanol and optionally other components, in a rectification system comprising at least two rectifying columns, two of which columns are operated at different pressures with the distillate from the column operated at lower pressure being fed to the column operated at higher pressure and a material stream being fed back from the column operated at higher pressure to the column operated at lower pressure, characterised in that the material stream taken

5

0 022 469

from the column operated at higher pressure is taken as a side stream from the distilling or concentrating portion and is led back to the column operated at lower pressure, and that a methanolrich stream, which contains practically all the methanol brought into the rectification system with the feed mixture, is withdrawn from the head of the column at higher pressure.

2. A process according to claim 1, characterised in that the first column is operated at atmospheric pressure and the second column is operated at a pressure of from 3 to 25 bars, preferably 10 to 15 bars.

3. A process according to claim 1 or 2, characterised in that a crude tetrahydrofuran which has been obtained by acid-catalysed reaction of impure butane-1,4-diol is fed to the process.

## Revendications

1. Procédé pour séparer en continu le méthanol de mélanges renfermant essentiellement du tétrahydrofuranne et, en outre, de l'eau, du méthanol et éventuellement aussi d'autres substances, dans une installation de rectification constituée par au moins deux colonnes de rectification dont deux au moins fonctionnent sous des pressions différentes, le produit de distillation provenant de la colonne qui fonctionne sous la plus faible pression étant amené à la colonne qui fonctionne sous une pression plus élevée, tandis qu'un courant de substance provenant de la colonne fonctionnant sous plus forte pression est renvoyé dans la colonne fonctionnant sous plus faible pression, ledit procédé étant caractérisé par le fait que le courant de substance prélevé de la colonne fonctionnant sous pression plus élevée est prélevé comme courant latéral de la partie d'entraînement ou de renforcement et est renvoyé dans la colonne fonctionnant sous plus faible pression, tandis qu'à la tête de la colonne fonctionnant sous plus forte pression, on soutire un courant de substance riche en méthanol qui renferme pratiquement la totalité du méthanol introduit avec la substance brute dans l'installation de rectification.

2. Procédé selon la revendication 1, caractérisé par le fait que la première colonne fonctionne sous la pression normale et que la seconde fonctionne sous une pression comprise entre 3 et 25 bar, de préférence entre 10 et 15 bar.

3. Procédé selon la revendication 1 ou 2, caractérisé par le fait qu'on utilise un tétrahydrofuranne qui a été obtenu par conversion catalytique acide à partir d'un butane-diol-(1,4) impur.

6